Europäisches Patentamt

(19) European Patent Office

Office européen des brevets

(11) Publication number: **0 299 430 B1**

(12) **EUROPEAN PATENT SPECIFICATION**

(45) Date of publication of patent specification :
**12.06.91 Bulletin 91/24**

(51) Int. Cl.$^5$ : **B01J 29/04**

(21) Application number : **88111138.9**

(22) Date of filing : **12.07.88**

(54) **Process for preparing a catalyst for the ammoximation of carbonylic compounds.**

(30) Priority : **14.07.87 IT 2126687**

(43) Date of publication of application :
**18.01.89 Bulletin 89/03**

(45) Publication of the grant of the patent :
**12.06.91 Bulletin 91/24**

(84) Designated Contracting States :
**AT BE DE ES FR GB IT NL SE**

(56) References cited :
EP-A- 0 077 522
EP-A- 0 111 700
EP-A- 0 208 311
EP-A- 0 267 362
FR-A- 2 471 950

(73) Proprietor : **MONTEDIPE S.r.l.**
**31, Foro Buonaparte**
**I-20121 Milan (IT)**

(72) Inventor : **Padovan, Mario**
**1 via Villa Mirabello**
**I-20125 Milan (IT)**
Inventor : **Roffia, Paolo, Dr.**
**25, via Valletta**
**I-21047 Saronno (Varese) (IT)**
Inventor : **De Alberti, Giordano**
**4, Largo Brianzoni**
**I-21010 Besnate (Varese) (IT)**

(74) Representative : **Weinhold, Peter, Dr.**
**Patentanwälte Dr. V. Schmied-Kowarzik**
**Dipl.-Ing. G. Dannenberg Dr. P. Weinhold Dr. D.**
**Gudel Dipl.-Ing. S. Schubert Dr. P. Barz**
**Siegfriedstrasse 8**
**W-8000 München 40 (DE)**

## Description

The invention relates to a process for preparing a silicon and titanium containing catalyst showing a zeolitic structure which is particularly suitable for the ammoximation of carbonylic compounds in trickle bed reactors.

From DE-A-1,245,371 it is known to obtain cyclohexanone oxime by catalytic reaction in the liquid phase of cyclohexanone with ammonia and hydrogen peroxide ("ammoximation reaction"), at a temperature from 5 to 40°C and with suitable reactant ratios in the presence of a catalyst consisting of phospho-tungstic acid or similar compounds. However, a drawback of this method is that this type of catalyst is difficult to handle, particularly during the separation of the product from the catalyst. EP-A-208,311 teaches that an effective alternative is the catalysis by a synthetic, crystalline, porous material based on silicon and titanium oxides (titanium-silicalite) ; in this connection see also US-A-4,410,501 and EP-A-132,550 and 190,609. It has also been found that a special type of titanium-silicalites (post-treated with $H_2O_2$) shows exceptional and astonishing catalytic properties in the ammoximation reaction of various carbonylic compounds (see EP-A-87 108 577) and that the thus activated titanium-silicalites can be used in continuous operations, both in an isothermal suspended-bed reactor, equipped with stirring means, and in an adiabatic trickle bed reactor. Titanium-silicalites can be prepared by starting from several titanium sources (e.g., tetra-ethyl ortho-titanate, tetra-isopropyl ortho-titanate or peroxy-titanates, optionally formed in situ etc.) and from several silicon sources (tetra-ethyl ortho-silicate, silica sol etc.) If the ammoximation process has to be accomplished in a trickle-bed reactor, a careful shaping of the catalyst is necessary. This can be done in further steps, such as pugging the titanium-silicalite with a suitable binding agent and then giving the mixture the shape of spheres, pellets, extrudates (optionally polylobate extrudates, having helical or non-helical grooves) etc. An object of the present invention is to provide a process which allows to obtain titanium-silicalite based catalysts directly in spherical, pellet or extrudate shape, in a much simpler way.

In its broadest aspect, the present invention relates to a process for preparing a catalyst comprising a synthetic crystalline, porous material based on silicon and titanium oxide, particularly suitable for the ammoximation of carbonylic compounds in trickle-bed reactors, characterized in that a preformed matrix of amorphous silica is impregnated with an aqueous solution containing a titanium compound and an organic nitrogen compound which acts as templating agent, and that the trus impregnated material is provided with a zeolitic structure by means of a conventional hydrothermal synthesis, i.e. a treatment at a temperature of from 150 to 200°C in an autoclave nudes autogenous pressure for 2 to 20 days, shape and size of the silica matrix being substantially preserved. The amorphous silica matrix may comprise spheres, extrudates (e.g. polylobate extrudates, optionally showing helical grooves) or silica pellets, in the normally available types and sizes. Silica types having a surface area of from 80 to 160 m²/g, a pore volume of from 0.5 to 1. 5 cm³/g, in the form of extrudates having a diameter from 1 to 10 mm, preferably from 3 to 6 mm, have proved to be particularly suitable.

As a titanium source, several water-soluble compounds can be used, such as e.g., alkyl titanates, $TiOCl_2$, peroxytitanates (optionally formed in situ), diisopropyl-bis-triethanolamine titanate and so forth ; the Si : Ti molar ratio in the catalyst is advantageously at least 30 and, preferably, 50. Tetrapropylammonium hydroxide can be considered as one of the most widely used templating compounds.

Very good results are obtained by impregnating preformed amorphous silica, dried at temperatures of from 100 to 350°C, according to the dry-impregnation technique, described e.g. in IND. ENG. CHEM. PROD. RED. DEV., Volume 20 (1981), page 441.

The thus impregnated preformed and solid material is transferred to an autoclave and is kept therein at a temperature of from 150 to 200°C preferably (approximately) 175°C and under its autogenous pressure, for from 2 to 20 days, preferably from 3 to 11 days. At the end of the hydrothermal synthesis, the solid material, which retains the shape and size of the silica used as the starting material, is washed with $H_2O$ up to neutral pH and is dried at e.g. 120-130°C for 15 hours. Thereafter it may optionally be calcined (for instance at 430°C for 10 hours).

The best yields are obtained by subsequently employing an activating washing step with an aqueous solution of hydrogen peroxide, optionally in the presence of an acid having a pK of equal to or lower than 5, preferably selected from sulphuric, phosphoric and hydrochloric acid, or in the presence of at least 10 kg of $NH_3$ per 100 kg of solution.

The catalysts prepared according to the invention were sucessfully used in continuous runs, for many hours, and with no indication of decay, for the ammoximation of various carbonylic compounds, such as e.g. acetone, cyclohexanone, methyl-ethyl-ketone (butan-2-one), acetophenone or cyclo-dodecanone, with $H_2O_2$ and $NH_3$. Particularly good results are obtained when the ammoximation is preceded by an activating washing of the catalyst, as disclosed e.g. in EP-A-87 108 577. The thus obtained catalyst can be used in trickle-bed reactors, provided with surfaces resistant to hydrogen peroxide.

The conversion to oxime can be carried out at a temperature of from 25 to 100°C (preferably of from 40 to 90°C, and particularly of from 60 to 90°C) ; tests carried out at 15°C gave poor results. The reaction can be carried out under atmospheric pressure, or under slightly higher pressure, in order to keep dissolved in the reaction medium at least the amount of ammonia which is required for the ammoximation. The $H_2O_2$ : carbonylic compound molar ratio generally ranges from 0.3 to 2.5 and preferably from 0.5 to 1.5. "$H_2O_2$" is intended to mean 100% pure hydrogen peroxide (dilution water excluded). The $NH_3$ : carbonylic compound molar ratio is equal to or higher than 1 (preferably 1.5), otherwise disturbing parallel reactions may take place. The reaction medium may comprise water or an organic solvent ; exceptionally good results were obtained by using as the solvent tert.-butyl alcohol and/or cyclohexanol, optionally mixed with dioxane or toluene. The tert.-butanol (and/or cyclohexanol) : carbonylic compound molar ratio should generally be from 0.1 to 100. Maintaining the hourly space velocity within the range of from 0.1 to 200 kg/hour (preferably from 2 to 200 kg/h) of ketone per kg of pure catalyst (binding agent excluded), and feeding the ketone as a mixture with the organic solvent e.g. tert.-butanol (and/or cyclohexanol), is recommended.

The following examples serve to illustrate the invention.

Example 1

(Syntheses of Titanium-Silicalite in Extrudate Form)

0.6 g of tetraisopropyl ortho-titanate were hydrolysed with 10 ml of deionized water under stirring and at room temperature, thus forming a white gel-like suspension, to which 20 ml of hydrogen peroxide (30% by weight) were added. To the resulting clear solution of orange colour there were added 30 ml of an aqueous tetrapropylammonium hydroxide solution (20% by weight) and the thus obtained clear solution was concentrated by evaporation to a volume of 20 ml. 30 g of extrudates (solid cylinders of 4 mm of diameter), consisting of amorphous silica having a surface area of 120 $m^2/g$ and a pore volume of 1.1 $cm^3/g$, were fed into a rotary flask kept under vacuum. The above solution was slowly poured onto the extrudate, while maintaining the vacuum inside the flask. The thus impregnated extrudates were transferred to an autoclave and subjected to a hydrothermal synthesis at 175°C for 9 days under autogenous pressure. At the end, after cooling, the obtained solid, still in the form of extrudates, was washed for an extended period of time to neutral pH with deionized water, dried at 120°C for 15 hours and then calcined at 430°C for 10h. The elemental analysis showed that the solid contained titanium, according to an Ti/(Ti + Si) atomic ratio = 0.45% ; the X-ray diffraction showed the presence of a crystalline product characterized by the typical reflections of titanium-silicalite such as those shown in US-A-4,410,501. The presence of titanium-silicalite was confirmed by the I.R. spectrum, where the typical bands at 960 and 550 cm⁻¹ were present (see the above U.S. patent) and by the nitrogen absorption isotherm (BET method).

Example 2

(Catalyst Activation with $H_2O_2$)

13 g of the product obtained in Example 1 were poured into an aqueous solution prepared from 26 ml of hydrogen peroxide (30% by weight) and 230 ml of diluted sulphuric acid (10% by weight) ; the resulting mixture was then stirred for 2 hours at 70°C ; thereafter the liquid was decanted. This activation was repeated once more with a fresh solution ; the extrudates were filtered off from the liquid and washed with deionized water (to neutral pH). The product was finally dried at 120°C for 15 hours and calcined at 550°C for 2 hours.

Example 3

2 g of the catalyst obtained according to Example 1 were fed into a trickle bed reactor charged with 12 ml/h of an organic solution (containing 8.67% by weight of cyclohexanone, 44.53% by weight of tert.-butyl alcohol and 40.80% by weight of water), with 0.66 ml/h of hydrogen peroxide (34% by weight) and with 0.4 litres/h of ammonia gas. At 80°C a cyclohexanone conversion of 35.5% and a corresponding oxime selectivity of 74.8% were obtained.

Example 4

Example 3 was repeated, the catalyst of Example 1 being replaced by the catalyst of Example 2 ; a cyclohexanone conversion of 37.6% and a corresponding oxime selectivity of 93.6% were obtained.

Example 5

5 g of the catalyst prepared according to Example 1 were fed into a trickle bed reactor charged with 30 ml/h of an organic solution (containing 8.10% by weight of cyclohexanone, 2.64% by weight of $H_2O_2$, 40.90% by weight of tert.-butyl alcohol and 48.36% by weight of water) and with 1 litre/h of ammonia gas. At 80°C a cyclohexanone conversion of 47.5%, and an oxime selectivity of 79.8% were observed.

Example 6

Example 1 was repeated, a different type of silica being subjected to impregnation, said silica being characterized by a surface area of 90 $m^2/g$ and by a pore volume of 0.6 $cm^3/g$, and being in the form of extruded cylinders (diameter 2 mm). For the impregnation there was used a solution which had been concentrated by evaporation to half-volume (with respect to the one of Example 1), the further impregnation procedure being maintained unchanged. The formation of titanium-silicalite was confirmed by X-ray diffraction spectroscopy, I.R. spectroscopy and nitrogen absorption (BET). Also this catalyst proved to be suitable for the ammoximation of carbonylic compounds in a trickle-bed reactor, yielding high oxime selectivities.

## Claims

1. Process for preparing a catalyst for the ammoximation of carbonylic compounds, said catalyst comprising a synthetic, crystalline, porous material based on silicon and titanium oxides, characterized in that a preformed matrix of amorphous silica is impregnated with an aqueous solution containing a titanium compound and an organic nitrogen compound which acts as a templating agent, and that the thus impregnated material is provided with a zeolitic structure by means of a treatment at a temperature of from 150 to 200°C in an autoclave under autogenous pressure for 2 to 20 days, whereby shape and size of the silica matrix are substantially retained.

2. Process according to claim 1, wherein said impregnation is a dry-impregnation.

3. Process according to any of the claims 1 and 2, wherein said matrix comprises extruded granules (extrudates).

4. Process according to claim 3, wherein said extruded granules have a polylobate shape, optionally showing helical grooves.

5. Process according to any of the preceding claims, wherein said thermal treatment in an autoclave is followed by a thermal treatment at a temperature of equal to or lower than 430°C, preferably 130°C, and by a subsequent activating washing step using an aqueous hydrogen peroxide solution.

6. Process according to claim 5, wherein said washing is carried out in the presence of an acid having a pK of equal to or lower than 5, preferably selected from sulphuric, phosphoric and hydrochloric acid.

7. Process according to claim 5, wherein said aqueous $H_2O_2$ solution contains at least 10 kg of $NH_3$ per 100 kg of solution.

8. Process according to any of the preceding claims, wherein the ratio of the silica source to the titanium source is such that the Si : Ti molar ratio, in the finished catalyst, is equal to or larger than 30, and, preferably, 50.

9. Process according to any of the preceding claims, wherein the titanium compound used as the starting material is selected from alkyl-titanates and peroxy-titanates, optionally formed in situ.

10. Catalyst for the ammoximation of carbonylic compounds, obtainable by the process of any of claims 1 to 9.

11. Use of the catalyst according to claim 10 for the ammoximation of carbonylic compounds, preferably selected from acetone, methyl-ethyl-ketone, cyclohexanone, cyclododecanone and acetophenone.

12. Use according to claim 11, wherein said catalyst is continuously exploited inside a trickle-bed reactor.

## Ansprüche

1. Verfahren zur Herstellung eines Katalysators zur Ammoximation von carbonylischen Verbindungen, der ein synthetisches, kristallines, poröses Material auf der Basis von Silicium- und Titanoxiden umfaßt, dadurch gekennzeichnet, daß eine vorgebildete Matrix aus amorphem Siliciumdioxid mit einer wäßrigen Lösung, die eine Titanverbindung und eine als Templat-bildendes Mittel wirkende organische Stickstoffverbindung enthält, imprägniert wird, und daß das so imprägnierte Material durch 2- bis 20-tägige Behandlung bei einer Temperatur von 150 bis 200°C in einem Autoklaven unter autogenem Druck eine Zeotlithstruktur erhält, wobei Form und Größe der Siliciumdioxid-Matrix im wesentlichen erhalten bleiben.

2. Verfahren nach Anspruch 1, bei welchem die Imprägnierung eine Trocken-Imprägnierung ist.

3. Verfahren nach irgendeinem der Ansprüche 1 und 2, bei welchem die Matrix stranggepreßte Körner (Extrudate) umfaßt.

4. Verfahren nach Anspruch 3, bei welchem die stranggepreßten Körner eine mehrlappige Form haben und gegebenenfalls spiralförmige Rillen aufweisen.

5. Verfahren nach irgendeinem der vorhergehenden Ansprüche, bei welchem der Wärmebehandlung in einem Autoklaven eine Wärmebehandlung bei einer Temperatur gleich oder niedriger als 430°C, vorzugsweise 130°C, und eine anschließende aktivierende Wäsche mit einer wäßrigen Wasserstoffperoxidlösung folgt.

6. Verfahren nach Aspruch 5, bei welchem die Wäsche in Anwesenheit einer Säure mit einem pK-Wert gleich oder niedriger als 5 durchgeführt wird, die vorzugsweise aus Schwefel-, Phosphor- und Salzsäure ausgewählt ist.

7. Verfahren nach Anspruch 5, bei welchem die wäßrige $H_2O_2$-Lösung mindestens 10 kg $NH_3$ pro 100 kg Lösung enthält.

8. Verfahren nach irgendeinem der vorhergehenden Ansprüche, bei welchem das Verhältnis von Siliciumdioxidquelle zur Titanquelle so bemessen ist, daß das Mol-Verhältnis von Si :Ti im fertigen Katalysator gleich oder größer als 30, vorzugsweise 50, ist.

9. Verfahren nach irgendeinem der vorhergehenden Ansprüche, bei welchem die als Ausgangsmaterial verwendete Titanverbindung aus Alkyltitanaten und Peroxytitanaten ausgewählt ist, die gegebenenfalls in situ gebildet werden.

10. Katalysator zur Ammoximation von carbonylischen Verbindungen, erhältlich nach einem Verfahren gemäß irgendeinem der Ansprüche 1 bis 9.

11. Die Verwendung des Katalysators gemäß Anspruch 10 zur Ammoximation von carbonylischen Verbindungen, vorzugsweise ausgewählt aus Aceton, Methylethylketon, Cyclohexanon, Cyclododecanon und Acetophenon.

12. Eine Verwendung gemäß Anspruch 11, bei welcher der Katalysator kontinuierlich in einem Rieselbett-Reaktor benutzt wird.

## Revendications

1. Procédé de préparation d'un catalyseur comprenant un matériau poreux cristallin synthétique à base de silicium et d'oxyde de titane, convenant particulièrement à l'ammoxymation de composés carbonyliques dans des réacteurs à lit et à écoulement descendant, caractérisé en ce qu'une matrice préformée, en silice amorphe, est imprégnée d'une solution aqueuse contenant un composé du titane et un composé de l'azote organique jouant le rôle d'un agent de modelage, et en ce que le matériau ainsi imprégné est pourvu d'une structure zéolitique grâce à une synthèse hydrothermale classique, c'est-à-dire un traitement à une température de 150 à 200°C dans un autoclave sous la pression spontanée pendant 2 à 20 jours, la forme et la taille de la matrice de silice étant pratiquement conservées.

2. Procédé selon la revendication 1, dans lequel ladite imprégnation est une imprégnation à sec.

3. Procédé selon l'une quelconque des revendications 1 et 2, dans lequel ladite matrice est constituée de granulés extrudés (extrudats).

4. Procédé selon la revendication 3, dans lequel lesdits granulés extrudés ont une forme polylobée, et présentant éventuellement des rainures hélicoïdales.

5. Procédé selon l'une quelconque des revendications précédentes, dans lequel ledit traitement thermique en autoclave est suivi d'un traitement thermique à une température inférieure ou égale à 430°C, de préférence de 130°C, et par une étape ultérieure de lavage d'activation faisant appel à une solution aqueuse d'eau oxygénée.

6. Procédé selon la revendication 5, dans lequel ledit lavage est mis en oeuvre en présence d'un acide ayant un pK inférieur ou égal à 5, de préférence choisi entre l'acide sulfurique, l'acide phosphorique et l'acide chlorhydrique.

7. Procédé selon la revendication 5, dans lequel ladite solution aqueuse de $H_2O_2$ contient au moins 10 kg de $NH_3$ par 100 kg de solution.

8. Procédé selon l'une quelconque des revendications précédentes, dans lequel le rapport de la source de silice à la source de titane est tel que la proportion molaire Si/Ti, dans le catalyseur fini, soit supérieure ou égale à 30, et de préférence égale à 50.

9. Procédé selon l'une quelconque des revendications précédentes, dans lequel le composé du titane utilisé comme matière de départ est choisi parmi les titanates d'alkyle et les peroxy-titanates, éventuellement formés in situ.

10. Catalyseur d'ammoxymation de composés carboxyliques, pouvant être obtenu par le procédé selon l'une quelconque des revendications 1 à 9.

11. Utilisation du catalyseur selon la revendication 10 pour l'ammoxymation de composés carbonyliques, de préférence choisis entre l'acétone, la méthyléthylcétone, la cyclohexanone, la cyclododécanone et l'acétophénone.

12. Utilisation selon la revendication 11, dans laquelle ledit catalyseur est utilisé en continu dans un réacteur à lit et à écoulement descendant.